# EUROPÄISCHE PATENTANMELDUNG

(11) **EP 4 014 946 A1**
(43) Veröffentlichungstag der Anmeldung: **22.06.2022**
(21) Anmeldenummer: 20000488.5
(22) Anmeldetag: 23.12.2020
(51) Int. Cl.: A61H 23/00, A61B 5/00, B25J 11/00, A61B 34/30

(54) **PATIENTENBEHANDLUNGSVORRICHTUNG UND VERFAHREN ZUR STEUERUNG EINER PATIENTENBEHANDLUNGSVORRICHTUNG**

(30) Priorität: 21.12.2020 EP 20216212
(71) Anmelder: Scheuerer, Joachim, 7450 Tiefencastel (CH)
(72) Erfinder: Scheuerer, Joachim, 7450 Tiefencastel (CH)
(74) Vertreter: Daub, Thomas

(57) **Zusammenfassung**

Die Erfindung geht aus von einer Pabentenbehandlungsvorrichtung (10) mit einer Patiententrageeinheit (12) für einen Patienten (14), mit einer ersten Behandlungseinheit (16) zur Behandlung eines ersten Körperbereichs (20) des Patienten (14) und mit zumindest einer zweiten Behandlungseinheit (18) zur Behandlung zumindest eines zweiten Körperbereichs (22) des Patienten (14).

Um eine gattungsgemäße Vorrichtung mit verbesserten Eigenschaften hinsichtlich einer Effizienz bereitzustellen, wird vorgeschlagen, dass die Patientenbehandlungsvomchtung (10) eine Steuereinheit (24) aufweist, welche in zumindest einem Betriebsmodus die erste Behandlungseinheit (16) und zumindest die zweite Behandlungseinheit (18) basierend auf Patientendaten des Patienten (14) jeweils individuell steuert.

## Beschreibung

### Stand der Technik

Die Erfindung betrifft eine Patientenbehandlungsvorrichtung nach dem Oberbegriff des Anspruchs 1, ein Verfahren zur Steuerung einer Patientenbehandlungsvorrichtung nach Anspruch 16, ein Computerprogrammprodukt nach Anspruch 17 und ein computerlesbares Medium nach Anspruch 18.

Aus dem Stand der Technik ist bereits eine Patientenbehandlungsvorrichtung, insbesondere ein Massagestuhl, bekannt. Hierbei erfolgt eine Behandlung eines Patienten unabhängig von Patientendaten eines Patienten.

Die Aufgabe der Erfindung besteht insbesondere darin, eine gattungsgemäße Vorrichtung mit verbesserten Eigenschaften hinsichtlich einer Effizienz bereitzustellen. Die Aufgabe wird erfindungsgemäß durch die Merkmale der Ansprüche 1, 16, 17 und 18 gelöst, während vorteilhafte Ausgestaltungen und Weiterbildungen der Erfindung den Unteransprüchen entnommen werden können.

### Vorteile der Erfindung

Die Erfindung geht aus von einer Patientenbehandlungsvorrichtung mit einer Patiententrageeinhelt für einen Patienten, mit einer ersten Behandlungseinheit zur Behandlung eines ersten Körperbereichs des Patienten und mit zumindest einer zweiten Behandlungseinheit zur Behandlung zumindest eines zweiten Körperbereichs des Patienten, wobei die Körperbereiche insbesondere in zumindest einem Betriebsmodus wesentlich voneinander beabstandet sind.

Es wird vorgeschlagen, dass die Patientenbehandlungsvorrichtung eine Steuereinheit aufweist, welche in zumindest dem Betriebsmodus die erste Behandlungseinheit und zumindest die zweite Behandlungseinheit, insbesondere gleichzeitig, basierend auf Patientendaten des Patienten jeweils individuell steuert.

Durch eine derartige Ausgestaltung kann eine Effizienz, Insbesondere eine Behandlungseffizienz und/oder eine Arbeitseffizienz, gesteigert werden. Mittels zumindest zweier Behandlungseinheiten können zumindest zwei Körperbereiche eines Patienten, insbesondere gleichzeitig, behandelt und damit eine Behandlung des Patienten effizienter, vorteilhaft schneller und/oder kompakter und/oder umfassender und/oder individueller, gestaltet werden. Damit können wiederum Behandlungskosten, insbesondere hinsichtlich einer Behandlungsdauer, gesenkt werden. Zudem kann ein Komfort, insbesondere ein Behandlungskomfort, vorteilhaft verbessert werden, und zwar indem eine Steuereinheit eine erste Behandlungseinheit und zumindest eine zweite Behandlungseinheit jeweils basierend auf Patientendaten des Patienten individuell steuert. Damit kann die Behandlung des Patienten individuell auf zu behandelnde Körperbereiche abgestimmt werden. Des Weiteren kann eine Flexibilität der Behandlung möglicherweise dahingehend gesteigert werden, dass mittels der Patientenbehandlungsvorrichtung unterschiedliche Behandlungsarten und/oder Therapien, insbesondere gleichzeitig, in einem ersten Körperbereich und zumindest einem zweiten Körperbereich, durchgeführt werden können. Somit kann die Patientenbehandlungsvorrichtung möglicherweise im Wellnessbereich, zur Massage und/oder zur Entspannung des Patienten, und/oder in der Schmerztherapie eingesetzt werden. Darüber hinaus kann die Patientenbehandlungsvorrichtung beispielsweise zur Prophylaxe des Patienten dienen, wobei insbesondere vorbeugend ein Verletzungsrisiko des Patienten minimiert werden kann. Insbesondere kann ein zwei- und/oder dreidimensionales Modell eines Bewegungsapparats des Patienten visualisiert und vorteilhaft zumindest ein zu behandelnder Teil des Bewegungsapparates visuell angezeigt werden, beispielsweise auf einem externen Gerät, insbesondere einem Mobilgerät und vorteilhaft einem Smartphone. Darüber hinaus wäre denkbar, dass mittels des zwei- und/oder dreidimensionalen Modells ein vorteilhaft schmerzfreier Zustand des Bewegungsapparats simuliert werden kann. Dies kann einen Patienten möglicherweise zu einer frühzeitigen Behandlung, vorzugsweise zur Prophylaxe, vorteilhaft beeinflussen. Durch die Behandlung mittels der Patientenbehandlungsvorrichtung kann dem Patienten ein verbessertes Körpergefühl und möglicherweise auch ein verbessertes Lebensgefühl, insbesondere ein zumindest teilweise und vorzugsweise vollständiges schmerzfreies Gefühl im Bewegungsapparat, ermöglicht werden. Durch die Behandlung von verhärteten und/oder schmerzenden und/oder blockierten Muskelbereichen des Patienten kann insbesondere eine körpereigene Produktion von Botenstoffen, insbesondere Myokinen, gesteigert werden.

Die Patientenbehandlungsvorrichtung ist, ohne darauf beschränkt zu sein, zur zumindest teilweisen Behandlung, beispielsweise medizinischen Behandlung, des Patienten vorgesehen. Die Behandlung könnte zu Wellnesszwecken und/oder zur Prävention und/oder zur Prophylaxe erfolgen. Alternativ und/oder zusätzlich könnte es sich bei der Behandlung um eine Therapie, insbesondere eine Körpertherapie und/oder eine Physiotherapie und/oder vorzugsweise um eine Schmerztherapie, und/oder eine Massage handeln. Es wäre denkbar, dass die Patientenbehandlungsvorrichtung eine eingriffsfreie Behandlung des Patienten ermöglicht.

Vorzugsweise ist die Patientenbehandlungsvorrichtung darauf ausgelegt, zumindest einen Teil eines Bewegungsapparats des Patienten, insbesondere zumindest einen Teil eines aktiven und/oder passiven Bewegungsapparats des Patienten, zu behandeln. Der Bewegungsapparat könnte beispielsweise zumindest einen Knochen und/oder zumindest einen Wirbelkörper und/oder zumindest ein Gelenk und/oder zumindest ein Band und/oder zumindest eine Sehne und/oder zumindest eine Faszie und/oder zumindest einen Muskel, insbesondere Muskelfasern und/oder Muskelgewebe, vorzugsweise einer Skelettmuskulatur, des Patienten aufweisen. Besonders bevorzugt ist die Patientenbehandlungsvorrichtung zur Behandlung zumindest eines Teils eines schmerzenden Bewegungsapparats, insbesondere zumindest einer verhärteten und/oder schmerzenden Sehne und/oder Faszie und/oder eines verhärteten und/oder schmerzenden Muskels, vorzugsweise von verhärteten und/oder schmerzenden und/oder verspannten Muskelbereichen des Patienten, insbesondere einer Myogelose des Patienten, vorgesehen. Vorteilhaft beschreibt die Myogelose eine Verdickung des Muskels mit kontraktilen Muskelbündeln, wobei eine derartige Verdickung des Muskels, welche beispielsweise druckschmerzempfindlich sein könnte, insbesondere in der Medizin auch unter einem sogenannten Trigger und/oder Triggerpunkt des Patienten bekannt ist. Die sich möglicherweise daraus ergebenden Krankheitsbilder werden beispielsweise als Fibromyalgie bzw. als Myofasciales Schmerzsyndrom bezeichnet. Die Patientenbehandlungsvorrichtung könnte den zu behandelnden Teil des Bewegungsapparats, insbesondere den Trigger, direkt und/oder zumindest einen Bereich des Bewegungsapparates. welcher an den zu behandelnden Teil zumindest abschnittsweise angrenzt, vorteilhaft gleichzeitig, behandeln.

Besonders bevorzugt umfassen die Patientendaten zumindest einen Behandlungsgrund, vorteilhaft Daten zu einem zu behandelnden Tell des insbesondere schmerzenden Bewegungsapparats des Patienten. Die Daten könnten eine Lage und/oder Ausrichtung und/oder Position des zu behandelnden Teils, vorteilhaft des Triggers, in dem Bewegungsapparat definieren. Vorzugsweise sind die Patientendaten individualisierte Patientendaten des Patienten. Insbesondere könnten die Patientendaten ein vorzugsweise individualisiertes zwei- und/oder dreidimensionales Modell des Bewegungsapparats des Patienten. insbesondere des gesamten Körpers des Patienten, umfassen. Das individualisierte Modell des Patienten könnte zumindest einen Teil und vorzugsweise ein vollständiges Skelettmodell des Patienten, ausgestattet mit zumindest einem Teil und vorzugsweise der gesamten Muskulatur und/oder Sehnen und/oder Faszien des Bewegungsapparats, aufweisen. Insbesondere ist das individualisierte Modell dazu vorgesehen, eine Lage und/oder Ausrichtung und/oder Position des individuell zu behandelnden Teils, vorteilhaft des individuellen Triggers, in dem Bewegungsapparat zu erfassen und/oder bevorzugt visuell darzustellen.

Die Patientenbehandlungsvorrichtung könnte eine Ausgabeeinheit zur vorzugsweisen visuellen Ausgabe des zwei- und/oder dreidimensionalen Modells aufweisen. insbesondere weist die Patientenbehandlungsvorrichtung eine Kommunikationseinheit zu einem Datentransfer zwischen der Steuereinheit und der Ausgabeeinheit auf. Die Kommunikationseinheit könnte beispielsweise zumindest eine Datenleitung, Insbesondere das Kabel, aufweisen oder als eine drahtlose Kommunikationseinheit ausgebildet sein. Die drahtlose Kommunikationseinheit könnte mittels Bluetooth und/oder RFID ein Signal und/oder Daten, vorzugsweise das individualisierte Modell, übertragen. Insbesondere könnte die Kommunikationseinheit mit einem externen Gerät, beispielsweise einem Handy, kommunizieren und/oder Daten austauschen. Möglicherweise könnte das externe Gerät die Ausgabeeinheit sein. Es wäre denkbar, dass einem Bediener, insbesondere einem Therapeuten und/oder einem Arzt und/oder dem Patienten, über beispielsweise eine App des Handys das individualisierte Modell visuell anzeigbar ist. Zusätzlich könnte der Bediener mittels des Handys eine Behandlung des zu behandelnden Teils, insbesondere basierend auf dem individualisieren Modell, steuern und/oder einstellen. Insbesondere in dem Betriebsmodus steuert die Steuereinheit die erste Behandlungseinheit und zumindest die zweite Behandlungseinheit, basierend auf dem zwei- und/oder dreidimensionalen Modell des Bewegungsapparats. Möglicherweise könnte die Patientenbehandlungsvorrichtung auch zu einer Diagnostik des Bewegungsapparats, insbesondere des zu behandelnden Teils des Bewegungsapparats, vorteilhaft zur Detektion der Patientendaten, eingesetzt werden. Es wäre denkbar, dass bei der Detektion der Patientendaten die Patientendaten unmittelbar mittels der Kommunikationseinheit zur Ausgabeeinheit übertragen und dem Bediener zur Verfügung gestellt werden.

Zur Präventionsbehandlung könnte zusätzlich denkbar sein, dass zumindest Standart-Bewegungsabläufe und/oder Haltungen des Bewegungsapparats mittels der Ausgabeeinheit über das individualisiere Modell animiert werden können. Zusätzlich könnten die Patientendaten beispielsweise ein Alter und/oder eine Größe und/oder ein Gewicht und/oder ein Geschlecht des Patienten beinhalten. Möglicherweise könnte eine Animation des individualisierten Modells unter Berücksichtigung beispielsweise des Alters erfolgen und besonders bevorzugt könnte ein zeitlicher Verlauf entlang einer Altersentwicklung des Bewegungsapparats simulierbar sein. Es wäre auch denkbar, dass eine Auswirkung der Behandlung auf den Bewegungsapparat, insbesondere eine Veränderung zumindest des Teils des Bewegungsapparats durch die Behandlung, simulierbar ist. Vorzugsweise könnte ein schmerzfreier Zustand des Bewegungsapparats simuliert werden.

Die Patientenbehandlungsvorrichtung könnte beispielsweise ein Gehäuse, insbesondere eine Kabine, aufweisen. Vorteilhaft ist das Gehäuse beweglich gelagert. Insbesondere begrenzt das Gehäuse einen innenraum, beispielsweise einen Behandlungsraum, In welchem die Patiententrageeinheit angeordnet ist Alternativ und/oder zusätzlich könnte sich die Patientenbehandlungsvorrichtung auch in einem Zimmer, insbesondere einem Gebäudezimmer und vorzugsweise einem Behandlungszimmer einer Arztpraxis und/oder einer Therapiepraxis und/oder in einer Wellnesspraxis und/oder möglicherweise in einer Wohnung des Patienten, befinden.

Die Patiententrageeinheit könnte beispielsweise eine Liege, insbesondere eine Patientenbehandlungsliege, und/oder ein Stuhl, insbesondere ein Patientenbehandlungsstuhl, sein. Vorteilhaft handelt es sich bei der Patiententrageeinheit um eine reversibel schwenkbare Patiententrageeinheit, insbesondere einen Variationsstuhl, welche zumindest eine von einer ersten Behandlungsposition der Patiententrageeinheit unterschiedliche zweite Behandlungsposition der Patiententrageeinheit aufweist, wobei die Patiententrageeinheit von der ersten Behandlungsposition in zumindest die zweite Behandlungsposition reversibel schwenkbar ist. Die Patiententrageeinheit könnte beispielsweise mechanisch und/oder manuell schwenkbar und/oder steuerbar sein. Insbesondere könnte die Steuereinheit die Patiententrageeinheit in zumindest einem Behandlungszustand steuern. Die erste Behandlungsposition oder die zweite Behandlungsposition könnte eine Sitzposition oder eine Liegeposition für den Patienten definieren. Alternativ und/oder zusätzlich könnte die Patiententrageeinheit höhenverstellbar sein. Denkbar wäre, dass die Patiententrageeinheit beweglich gelagert ist und insbesondere zumindest eine Rolleinheit zur Bewegung der Patiententrageeinheit, vorteilhaft in dem Raum und/oder dem Behandlungszimmer, aufweist. Die Rolleinheit könnte an einem Fuß der Patiententrageeinheit angeordnet sein. Vorzugsweise stellt die Patiententrageeinheit in zumindest dem Behandlungszustand eine zumindest im Wesentlichen eckige oder runde, und vorteilhaft ovale Liegefläche für den Patienten bereit. In dem Behandlungszustand könnte sich der Patient auf der Patiententrageeinheit sitzend und/oder liegend, vorteilhaft auf der Liegefläche, befinden.

Der erste Körperbereich und/oder der zweite Körperbereich könnte/könnten zumindest teilweise beispielsweise ein Kopf- und/oder ein Schulter- und/oder ein Rücken- und/oder ein Arm- und/oder ein Bein- und/oder ein Brust- und/oder ein Bauchbereich sein. Insbesondere unterscheidet sich der erste Körperbereich von dem zweiten Körperbereich. Beispielsweise könnte es sich in zumindest dem Betriebsmodus bei dem ersten Körperbereich um den Rückenbereich und bei dem zweiten Körperbereich um den Beinbereich handeln. Alternativ könnten der erste Körperbereich und der zweite Körperbereich zumindest jeweils einen Teilbereich eines Bereichs des Patienten, insbesondere eines der zuvor genannten Bereiche, definieren, welche jedoch vorzugsweise in zumindest dem Betriebsmodus wesentlich voneinander beabstandet sind. Es wäre denkbar, dass in dem Betriebsmodus der erste Körperbereich einen rechten Schulterbereich des Patienten und der zweite Körperbereich einen linken Schulterbereich des Patienten definiert. Vorzugsweise sind der erste Körperbereich und der zweite Körperbereich in dem Betriebsmodus wesentlich voneinander beabstandet, insbesondere um zumindest 0,5 cm, vorteilhaft um zumindest 1 cm, vorzugsweise um zumindest 5 cm und insbesondere um höchstens 2 m, vorteilhaft um höchstens 1 m und besonders bevorzugt um höchstens 30 cm.

Die erste Behandlungseinheit und/oder zumindest die zweite Behandlungseinheit könnten den Patienten in einer Sitzposition des Patienten und vorteilhaft in einer Liegeposition des Patienten auf der Liegefläche behandeln. In dem Behandlungszustand könnte sich die Patientenbehandlungsvorrichtung in einem Betriebszustand befinden. Der Betriebszustand könnte mehrere Betriebsmodi aufweisen. In zumindest dem Betriebsmodus ist die Steuereinheit dazu vorgesehen, die erste Behandlungseinheit und zumindest die zweite Behandlungseinheit basierend auf Patientendaten jeweils individuell zu steuern. Beispielsweise könnte die Steuereinheit in dem Betriebsmodus die erste Behandlungseinheit und die zweite Behandlungseinheit gleichzeitig steuern. Alternativ könnte die Steuereinheit in dem Betriebsmodus die erste Behandlungseinheit oder die zweite Behandlungseinheit steuern und in einem weiteren Betriebsmodus der Betriebsmodi die erste Behandlungseinheit und die zweite Behandlungseinheit gleichzeitig steuern.

Vorzugsweise erfolgt die Behandlung mittels der Patientenbehandlungsvorrichtung in dem Behandlungszustand zumindest teilweise und vorzugsweise vollständig automatisch. Der Bediener, insbesondere der Therapeut und/oder der Arzt, könnte die Behandlung, vorzugsweise die Behandlung, starten und/oder beenden und/oder vorzugsweise Einstellungen zur Behandlung vornehmen. Es wäre jedoch auch denkbar, dass der Patient der Bediener ist und die Patientenbehandlungsvorrichtung selbst bedient.

Die Steuereinheit könnte eine Zentralsteuereinheit der Patientenbehandlungsvorrichtung sein. Die Zentralsteuereinheit könnte in dem Betriebszustand die erste Behandlungseinheit und/oder zumindest die zweite Behandlungseinheit zentral steuern. Alternativ wäre denkbar, dass die Steuereinheit eine erste Teilsteuereinheit der ersten Behandlungseinheit und/oder zumindest eine zweite Teilsteuereinheit der zweiten Behandlungseinheit aufweist, wobei vorteilhaft die erste Teilsteuereinheit in der ersten Behandlungseinheit und/oder die zweite Teilsteuereinheit in der zweiten Behandlungseinheit angeordnet sein könnte/könnten. Insbesondere sind die erste Teilsteuereinheit und die zweite Teitsteuereinheit zumindest elektrisch miteinander verbunden. Denkbar wäre auch, dass die erste Teilsteuereinheit und/oder die zweite Teilsteuereinheit die gesamte Steuereinheit bildet/bilden.

Die Steuereinheit, beispielsweise ein Computer, könnte zumindest eine Recheneinheit und zusätzlich zu der Recheneinheit zumindest eine Speichereinheit aufweisen, in weleher zumindest eine Software, insbesondere ein Steuer- und/oder Regel- und/oder Kontrollprogramm, vorteilhaft zumindest ein Behandlungsprogramm, gespeichert sein könnte, welches zu einer Ausführung durch die Recheneinheit vorgesehen ist. Alternativ und/oder zusätzlich könnte es sich bei der Speichereinheit um einen externen Datenträger, belspielsweise eine CD-ROM und/oder einen USB-Stick, und/oder eine externe Datenbank handeln, auf welche die Steuereinheit, insbesondere mittels einer drahtlosen Verbindung, beispielsweise WLAN und/oder Bluetooth, zugreifen könnte. Die Speichereinheit könnte vorteilhaft unterschiedliche Behandlungsprogramme zur Behandlung des Patienten der Steuereinheit bereitstellen. In dem Betriebszustand könnte die Steuereinheit zumindest das Behandlungsprogramm steuern und/oder regeln.

Möglicherweise könnte das Behandlungsprogramm, insbesondere eine Steuerung und/oder Regelung der ersten Behandlungseinheit und der zweiten Behandlungseinheit zur Behandlung des Patienten, bereits vordefiniert und/oder voreingestellt sein. Vorzugsweise ist das Behandlungsprogramm, und zwar insbesondere die Steuerung der ersten Behandlungseinheit und/oder der zweiten Behandlungseinheit, mittels der Patientendaten individuell an den Patienten anpassbar. Die Patientendaten könnten bereits, insbesondere in der Speichereinheit, gespeichert sein. In dem Betriebszustand könnte die Steuereinheit auf die Speichereinheit zugreifen und die erste Behandlungseinheit und die zweite Behandlungseinheit basierend auf den Patientendaten jeweils individuell steuern. Alternativ und/oder zusätzlich könnte der Bediener die Patientendaten über eine Bedienerschnittstelle der Patientenbehandlungsvorrichtung der Steuereinheit übermitteln und/oder manuell eingeben und/oder in einem von der Steuereinheit bereitgestellten Auswahlmenü manuell auswählen.

Die Bedienerschnittstelle kann leitungsgebunden, insbesondere elektrisch und/oder optisch, und/oder drahtlos mit der Steuereinheit verbunden sein. Die Bedienerschnittstelle könnte an der ersten Behandlungseinheit und/oder der zweiten Behandlungseinheit angeordnet und/oder in die Patiententrageeinheit eingelassen sein. Alternativ und/oder zusätzlich könnte die Bedienerschnittstelle durch ein Sichtfenster der Patiententrageeinheit und/oder der ersten Behandlungseinheit und/oder der zweiten Behandlungseinheit hindurch sichtbar angeordnet sein. Die Bedienerschnittstelle könnte zu einer optischen und/oder akustischen und/oder haptischen Kommunikation zwischen dem Bediener und der Steuereinheit vorgesehen sein. Vorzugsweise ist die Bedienerschnittstelle zu einer Eingabe und/oder zu einer Auswahl zumindest eines Betriebsparameters vorgesehen. Zumindest ein Betriebsparameter könnte beispielsweise das Behandlungsprogramm und/oder die Patientendaten sein. Alternativ und/oder zusätzlich könnte der Betriebsparameter zumindest eine Einstellung des Behandlungsprogramms, wie beispielsweise eine Behandlungsdauer und/oder eine Wiederholungsanzahl der Behandlung, sein. Ferner könnte die Bedienerschnittstelle zu einer Ausgabe zumindest einer optischen und/oder akustischen Bedienerinformation an den Bediener vorgesehen sein. Vorteilhaft informiert die Bedienerschnittstelle den Bediener bei einem Beginn und/oder einer Beendigung und/oder einer Änderung der Behandlung, insbesondere des Behandlungsprogramms.

Möglicherweise könnte die Steuereinheit sprachgesteuerte Befehle des Bedieners und/oder die Patientendaten akustisch von dem Bediener empfangen und/oder verarbeiten und/oder an die erste Behandlungseinheit und/oder die zweite Behandlungseinheit weiterleiten, und/oder bevorzugt die Steuerung der ersten Behandlungseinheit und/oder der zweiten Behandlungseinheit anpassen und/oder verändern.

In der vorliegenden Anmeldung dienen Zahlwörter, wie beispielsweise "erste/r/s" und "zweite/r/s", welche bestimmten Begriffen vorangestellt sind, lediglich zu einer Unterscheidung von Objekten und/oder einer Zuordnung zwischen Objekten untereinander und implizieren keine vorhandene Gesamtanzahl und/oder Rangfolge der Objekte. Insbesondere impliziert ein "zweites Objekt" nicht zwangsläufig ein Vorhandensein eines "ersten Objekts". Des Weiteren soll hier und im Folgenden unter "vorgesehen" speziell programmiert, ausgelegt und/oder ausgestattet verstanden werden. Darunter, dass ein Objekt zu einer bestimmten Funktion vorgesehen ist, soll verstanden werden, dass das Objekt diese bestimmte Funktion in zumindest einem Anwendungs- und/oder Betriebszustand erfüllt und/oder ausführt.

Ferner wird vorgeschlagen, dass die erste Behandlungseinheit und/oder die zweite Behandlungseinheit zur Behandlung zumindest eines Teils des Bewegungsapparats des Patienten im ersten Körperbereich und/oder im zweiten Körperbereich eine Bearbeitungseinheit mit zumindest einem Bearbeitungskopf aufweist/aufweisen. Hierdurch kann eine effiziente und/oder patientengerechte Behandlung, insbesondere patientenfreundliche Behandlung, ermöglicht werden. Zudem kann mit einer Bearbeitungseinheit, welche zumindest einen Bearbeitungskopf aufweist, eine Behandlung an den Patienten angepasst und damit ein Behandlungskomfort verbessert werden.

Vorteilhaft ist die erste Behandlungseinheit und/oder die zweite Behandlungseinheit dazu vorgesehen, unterschiedliche Behandlungen und/oder Therapien des Patienten, insbesondere im ersten Körperbereich und/oder im zweiten Körperbereich, zu ermöglichen. Insbesondere ist die Bearbeitungseinheit zur Durchführung zumindest der Behandlung vorgesehen. In dem Betriebsmodus steuert die Steuereinheit die Bearbeitungseinheit. Der Bearbeitungskopf könnte möglicherweise an die jeweilige Behandlung anpassbar sein. In dem Behandlungszustand und in dem Betriebszustand, insbesondere in dem Betriebsmodus, kontaktiert der Bearbeitungskopf den Patienten im ersten Körperbereich und/oder im zweiten Körperbereich. Der Bearbeitungskopf könnte auf einer Oberfläche des Patienten, beispielsweise einer Haut und/oder einer Kleidung, aufliegen. Zusätzlich könnte der Bearbeitungskopf einen Druck, insbesondere Anpressdruck, auf den Patienten im ersten Körperbereich und/oder im zweiten Körperbereich ausüben.

Es wäre denkbar, dass der Bearbeitungskopf fest und insbesondere werkzeuglos unlösbar mit der ersten Behandlungseinheit und/oder der zweiten Behandlungseinheit verbunden Ist. Insbesondere ist der Bearbeitungskopf dazu vorgesehen, lediglich eine Art von Behandlung, insbesondere das Behandlungsprogramm, auszuführen. Um jedoch eine Flexibilität und eine Effizienz einer Behandlung weiter zu steigern, wird vorgeschlagen, dass die Bearbeitungseinheit eine Kopplungseinheit und einen Satz von Bearbeitungsköpfen aufweist, welche untereinander austauschbar und mit der Kopplungseinheit verbindbar sind. Zudem kann mittels der Kopplungseinheit ein vorteilhaft komfortabler und/oder schneller und/oder leichter Austausch der Behandlungsköpfe bereitgestellt werden.

Die Kopplungseinheit könnte den Bearbeitungskopf in einem montierten Zustand zumindest kraft- und/oder formschlüssig, beispielsweise mit einer Steckverbindung und/oder einer Drehverbindung und/oder einer Schraubverbindung und/oder einer Rastverbindung, fixieren. Vorzugsweise ist der Bearbeitungskopf reversibel, insbesondere zerstörungsfrei, mit der Kopplungseinheit verbindbar und/oder von der Kopplungseinheit lösbar. Es wäre denkbar, dass der Bediener den Bearbeitungskopf manuell auswechselt. Alternativ und/oder zusätzlich könnte der Bediener über die Bedienerschnittstelle den Bearbeitungskopf aus dem Satz von Behandlungsköpfen auswählen. Möglicherweise könnte/könnten die erste Behandlungseinheit und/oder die zweite Behandlungseinheit dazu vorgesehen sein, den Bearbeitungskopf selbstständig, insbesondere automatisch, auszutauschen und/oder zu wechseln. Die Steuereinheit könnte einen Austausch des Bearbeitungskopfes steuern und/oder regeln.

Vorzugsweise weist die Bearbeitungseinheit eine Lagereinheit, insbesondere ein Magazin, zur Lagerung des Satzes von Bearbeitungsköpfen, insbesondere in der Bearbeitungseinheit, auf. Die Lagereinheit könnte mit der Kopplungseinheit verbunden, insbesondere einstückig und vorzugsweise einteilig ausgebildet, sein. In dem Betriebszustand könnte die Steuereinheit die Lagereinheit zum Austausch des Behandlungskopfes steuern und/oder regeln. Die Steuereinheit könnte die Lagereinheit derart ansteuern, dass nach einer Auswahl des Behandlungsprogramms, insbesondere durch den Bediener, die Lagereinheit solange den Satz der Bearbeitungsköpfe durchläuft, bis ein für die Behandlung entsprechender Bearbeitungskopf gefunden ist. Der für die Behandlung entsprechende Behandlungskopf könnte dem Patienten zur Behandlung bereitstellbar sein und insbesondere könnte die Steuereinheit den für die Behandlung entsprechenden Behandlungskopf mit der Kopplungseinheit verbinden.

Unter "einstückig" soll zumindest stoffschlüssig verbunden verstanden werden, beispielsweise durch einen Klebeprozess, einen Anspritzprozess und/oder einen anderen, einem Fachmann als sinnvoll erscheinenden Prozess, und/oder in einem Stück geformt verstanden werden, wie beispielsweise durch eine Herstellung in einem Ein- oder Mehrkomponentengussverfahren und/oder durch eine Herstellung aus einem Guss und/oder in einem Stanzverfahren aus einem einzelnen Rohling. Unter "einteilig" soll insbesondere in einem Stück geformt verstanden werden. Vorzugsweise wird dieses eine Stück aus einem einzelnen Rohling, einer Masse und/oder einem Guss, besonders bevorzugt in einem Spritzgussverfahren, insbesondere einem Ein- und/oder Mehrkomponenten-Spritzgussverfahren, und/oder in einem 3D-Druckgussverfahren hergestellt.

Zur Durchführung einer Wellentherapie, insbesondere einer Stoßwellentherapie, im ersten Körperbereich und/oder im zweiten Körperbereich wird vorgeschlagen, dass die Bearbeitungseinheit zumindest einen Wellentherapiekopf aufweist. Dadurch kann eine Flexibilität, hinsichtlich einer Behandlung, insbesondere einer Anregungstherapie, gesteigert werden. Durch einen als Wellentherapiekopf ausgebildeten Bearbeitungskopf kann die Behandlung effizienter und komfortabler durchgeführt werden. Vorzugsweise kann damit eine Wellentherapie, insbesondere eine Stoßwellentherapie, für den Patienten bereitgestellt werden, welche vorteilhaft eine zielgerichtete und/oder schonende Behandlung eines Bewegungsapparats des Patienten, besonders bevorzugt eines Triggers des Patienten, ermöglichen kann. Des Weiteren können unterschiedliche Wellenbehandlungen, beispielsweise mit Schall und/oder Druck und/oder (Laser)-Licht und/oder Magnetismus und/oder elektrischem Strom und/oder Wärme, durchgeführt werden. Darüber hinaus kann möglicherweise mittels der Wellentherapie eine Wundheilung des Patienten stimuliert werden. Zudem kann bei zumindest einem Knochenbruch die Wellentherapie Knochenzellbildung anregen und/oder lokale Durchblutungsstörungen zumindest teilweise und vorzugsweise vollständig auflösen.

Der Wellentherapiekopf könnte Wellen, beispielsweise Schallwellen, insbesondere Ultraschallwellen, und/oder Stoßwellen, insbesondere Druckwellen, und/oder Infrarotstrahlung und/oder Mikrowellen, erzeugen und/oder aussenden. Der Wellentherapiekopf könnte beispielsweise ein Schallkopf und/oder ein Infrarottherapiekopf und/oder ein Mikrowellentherapiekopf sein. Die Steuereinheit könnte In dem Betriebsmodus die Wellentherapie über den Wellentherapiekopf im ersten Körperbereich und/oder im zweiten Körperbereich steuern. Die Stoßwellentherapie könnte beispielsweise eine extrakorporale Stoßwellentherapie (ESW) und/oder eine Triggerpunkt Stoßwellentherapie (TPST) sein. Bei dem zuvor genannten Betriebsparameter könnte es sich beispielsweise um einen Betriebsparameter der Wellentherapie handeln. Beispielsweise ist der Betriebsparameter der Wellentherapie, insbesondere eine Grundeinstellung der Wellentherapie, bereits voreingestellt. Alternativ und/oder zusätzlich könnte der Bediener den Betriebsparameter, wie beispielsweise einen Druck des Wellentherapiekopfs und/oder eine Bewegungsrichtung des Wellentherapiekopfs und/oder einen Druckimpuls des Wellentherapiekopfs, manuell einstellen.

Zur Behandlung mittels der Wellentherapie könnte die Patientenbehandlungsvorrichtung ein Gel, insbesondere ein wasserhaltiges Gel, und/oder ein Öl, beispielsweise ein Rizinusöl, aufweisen, mittels welchem die Wellen auf den Patienten, insbesondere in den Bewegungsapparat, übertragbar sind.

Vorzugsweise ist der Wellentherapiekopf zur radialen Stoßwellentherapie vorgesehen. Dadurch kann insbesondere eine radiale Oberflächenbehandlung eines Bewegungsapparats eines Patienten bereitgestellt werden. Zudem kann im Gegensatz zu einer fokussierten Stoßwellentherapie während einer Behandlung ein größerer Zielbereich eines zu behandelnden Teils des Bewegungsapparats behandelt werden. Der Wellentherapiekopf könnte beispielsweise zur Aussendung und/oder Erzeugung einer radialen Stoßwelle, insbesondere einer radialen Druckwelle, ein druckluftbeschleunigendes Projektil aufweisen. In dem Betriebsmodus könnte die von dem Wellentherapiekopf ausgehende Stoßwelle ein Gewebe des Patienten, insbesondere den Bewegungsapparat, zumindest teilweise durchdringen. Insbesondere übt der Wellentherapiekopf bei der radialen Stoßwellentherapie einen Anpressdruck auf den Patienten, insbesondere in dem ersten Körperbereich und/oder dem zweiten Körperbereich, aus. Die Steuereinheit könnte den Wellentherapiekopf zu unterschiedlichen zweidimensionalen und/oder dreidimensionalen Bewegungen, wie walken und/oder kreisen, ansteuern.

Alternativ und/oder zusätzlich wird vorgeschlagen, dass der Wellentherapiekopf zur fokussierten Stoßwellentherapie vorgesehen ist. Damit kann eine besonders effiziente Wellentherapie, Insbesondere Stoßwellentherapie, bereitgestellt werden, wobei gerade tiefliegende zu behandelnde Teile eines Bewegungsapparats, insbesondere Trigger, eines Patienten behandelt werden können. Zudem kann bei einer fokussierten Stoßwellentherapie auf ein Anpressdruck am Patienten verzichtet werden. Darüber hinaus können mit der fokussierten Stoßwellentherapie besonders kurze Stoßwellen mit hoher Energie zu einer gezielten und/oder punktuellen Behandlung des Bewegungsapparats, insbesondere des Triggers, genutzt werden, da die Stoßwellen bei der Behandlung punktuell auf den zu behandelnden Teil des Bewegungsapparats, insbesondere den Trigger, ausgerichtet werden.

Die fokussierte Stoßwellentherapie könnte, vorteilhaft im Gegensatz zu der radialen Stoßwellentherapie, zur Behandlung von zumindest einem tiefliegenden zu behandelnden Teil des Bewegungsapparats einsetzbar sein. Es wäre denkbar, dass sich der tiefliegende zu behandelnde Teil des Bewegungsapparats insbesondere zumindest 1 cm, vorteilhaft zumindest 3 cm und besonders vorteilhaft zumindest 5 cm unterhalb der Oberfläche des Patienten, insbesondere der Haut, befindet.

In einem weiteren Aspekt der Erfindung wird vorgeschlagen, dass die Bearbeitungseinheit zumindest einen Massagekopf zur Durchführung einer Massage im ersten Körperbereich und/oder im zweiten Körperbereich aufweist. Dadurch kann eine Vielseitigkeit einer Behandlung eines Patienten weiter gesteigert werden.

Die Massage könnte möglicherweise zum Entspannen und/oder Aufwärmen des Bewegungsapparats, insbesondere der Skelettmuskulatur, vorgesehen sein. Denkbar wäre, dass die Steuereinheit in dem Betriebsmodus die Bearbeitungseinheit zur Durchführung der Massage steuert. In dem weiteren Betriebsmodus, welcher insbesondere nach der Durchführung der Massage einsetzt, könnten die erste Behandlungseinheit und/oder die zweite Behandlungseinheit die Behandlung mittels der Wellentherapie fortsetzen. So könnte die Steuereinheit in dem weiteren Betriebsmodus die Wellentherapie steuern. Alternativ könnte in dem Betriebsmodus die Wellentherapie und in dem weiteren Betriebsmodus die Massage, insbesondere zur Entspannung, durchführbar sein.

Der als Massagekopf ausgebildete Bearbeitungskopf könnte beispielsweise zumindest eine insbesondere bewegliche Massagekugel und/oder eine Massagenadel und/oder eine Massagepistole aufweisen. Der Massagekopf könnte zur mechanischen Beeinflussung des Bewegungsapparats, insbesondere von Haut und/oder Bindegewebe und/oder Muskulatur, insbesondere der Skelettmuskulatur, durch Dehnungs- und/oder Zug- und/oder Druckreiz vorgesehen sein. Insbesondere übt der Massagekopf in dem Betriebsmodus einen Anpressdruck auf den Patienten aus und massiert zumindest den ersten Körperbereich und/oder den zweiten Körperbereich, insbesondere zumindest den Bewegungsapparat.

Um eine Effizienz und/oder eine Flexibilität, hinsichtlich einer Behandlung, weiter zu steigen, wird vorgeschlagen, dass die Bearbeitungseinheit einen Detektionskopf zur Detektion zumindest des zu behandelnden Teils des Bewegungsapparats, insbesondere eines latenten Schmerzpunkts, des Patienten aufweist Zudem kann die Effizienz insbesondere hinsichtlich einer Arbeits- und/oder Kosteneffizienz weiter gesteigert und vorteilhaft ein Behandlungsaufwand und/oder Behandlungskosten minimiert werden. Darüber hinaus kann beispielsweise eine Elastographie des Patienten ermöglicht werden. Außerdem kann ein Behandlungszustand und/oder ein Behandlungsfortschritt des Pateinten erfasst und/oder dokumentiert und/oder aufgezeichnet werden.

Die Detektion des zu behandelnden Teils des Bewegungsapparats, insbesondere der Patientendaten, könnte in dem Betriebsmodus erfolgen. Die Steuereinheit könnte möglicherweise in dem Betriebsmodus die erste Behandlungseinheit zur Detektion, insbesondere zur Diagnostik, und/oder die zweite Behandlungseinheit zur Behandlung, insbesondere zur Massage, vorteilhaft gleichzeitig, steuern. Vorzugsweise ist die Bearbeitungseinheit mit dem Detektionskopf, ohne darauf beschränkt zu sein, zur Elastographie, insbesondere zur Ultraschallmessung einer Gewebesteifigkeit und/oder Gewebeelastizität des Bewegungsapparats des Patienten, vorgesehen.

Insbesondere ist der Bearbeitungskopf als Detektionskopf ausgebildet. Der Detektionskopf könnte beispielsweise ein weiterer Schallkopf, insbesondere ein weiterer Ultraschallkopf, und/oder möglicherweise der zuvor genannte Schallkopf sein. In dem Behandlungszustand und in dem Betriebszustand könnte der Bediener den Detektionskopf manuell über zumindest einen Teil des Körpers des Patienten führen. Es wäre auch denkbar, dass die Steuereinheit den Detektionskopf zur Detektion, insbesondere zur Elastographie, in dem Betriebsmodus steuert und der Detektionskopf den Körper des Patienten automatisch entlangfährt. Der Detektionskopf könnte die Patientendaten, vorzugsweise den zu behandelnden Teil des Bewegungsapparats, insbesondere den Trigger, detektieren und/oder ermitteln. Insbesondere könnte mittels des Detektionskopf das zwei- und/oder dreidimensionale Modell des Bewegungsapparats erstellbar sein. Vorteilhaft könnte der in dem Betriebsmodus detektierte zu behandelnde Teil des Bewegungsapparats unmittelbar in dem weiteren Betriebsmodus behandelt werden.

Damit ein Bearbeitungskopf vorteilhaft in eine besonders behandlungsgünstige Position gebracht werden kann, wodurch wiederum neben einem Behandlungskomfort auch eine Flexibilität einer Behandlung, hinsichtlich einer Behandlungslage, verbessert werden kann, wobei die Behandlungslage individuell auf einen zu behandelnden Teil eines Bewegungsapparats eines Patienten, angepasst werden kann, wird vorgeschlagen, dass die erste Behandlungseinheit und/oder die zweite Behandlungseinheit eine Transporteinheit aufweist/aufweisen, mittels welcher zumindest der Bearbeitungskopf in eine auf den Patientendaten basierende Behandlungslage bringbar ist.

In zumindest dem Betriebsmodus könnte die Steuereinheit die Transporteinheit steuern, insbesondere justieren, und zwar könnte die Transporteinheit vorteilhaft von der Steuereinheit zumindest einen Steuerbefehl empfangen und/oder ausführen. Alternativ und/oder zusätzlich könnte der Bediener die Transporteinheit zur Behandlung des ersten Körperbereichs und/oder des zweiten Körperbereichs manuell und/oder über die Bedienerschnittstelle justieren. Die Behandlungslage definiert insbesondere eine auf den Patientendaten basierende individualisierte Lage und/oder Position und/oder Ausrichtung des Bearbeitungskopfes. In dem Behandlungszustand könnte sich der Bearbeitungskopf in der Behandlungslage befinden und vorteilhaft die Behandlung des Patienten ermöglichen. Vorzugsweise berührt der Bearbeitungskopf in der Behandlungslage den Patienten, insbesondere in dem ersten Körperbereich und/oder dem zweiten Körperbereich.

Die Transporteinheit könnte eine hochsensible Transporteinheit sein, welche insbesondere zumindest Dezimeter genau, vorteilhaft zumindest Zentimeter genau, vorzugsweise Millimeter genau und besonders bevorzugt Mikrometer genau, insbesondere mit der Steuereinheit, justierbar ist. In einer besonders bevorzugten Ausführung weist die Transporteinheit einen Roboterarm mit zumindest einem Gelenk auf. Damit kann eine besonders bewegliche und/oder flexible Behandlungseinheit bereitgestellt werden, welche insbesondere mittels des Gelenks in eine an einen Patienten individuell angepasste Behandlungslage gebracht werden kann.

Der Roboterarm könnte, ohne darauf beschränkt zu sein, als ein Medizinal-Roboterarm ausgebildet sein, welcher insbesondere eine feinfühlige Sensorik aufweist. Das Gelenk könnte beispielsweise ein Drehgelenk und/oder ein Sattelgelenk und/oder ein Eigelenk und/oder ein Zapfengelenk und/oder ein Zylindergelenk, insbesondere ein Scharniergelenk, und vorteilhaft ein Kugelgelenk sein. Insbesondere ist der Roboterarm mittels des Gelenks um insbesondere zumindest 80°, vorteilhaft zumindest 170°, vorzugsweise um zumindest 280°, besonders vorteilhaft um zumindest 350° beweglich gelagert, insbesondere drehbar.

Die Patiententrageeinheit könnte in mehrere Bereiche eingeteilt sein, welche insbesondere eine Ausrichtung für den Patienten vorgeben und/oder definieren. Vorzugsweise weist die Patiententrageeinheit einen Oberkörperbereich und/oder einen Unterkörperbereich auf. Insbesondere befindet sich in dem Behandlungszustand ein Oberkörper des Patienten, insbesondere zumindest ein Kopf und/oder eine Schulter, in dem Oberkörperbereich der Patiententrageeinheit. Alternativ und/oder zusätzlich könnte sich ein Unterkörper des Patienten, insbesondre zumindest ein Bein und/oder ein Fuß, in dem Unterkörperbereich befinden. In dem Oberkörperbereich könnte die Patiententrageeinheit beispielsweise ein Kissen oder eine Aussparung für einen Kopf des Patienten aufweisen. Alternativ und/oder zusätzlich könnte die Patiententrageeinheit eine Aussparung für zumindest einen Fuß des Patienten im Unterkörperbereich aufweisen.

Es wird vorgeschlagen, dass die erste Behandlungseinheit in dem Oberkörperbereich der Patiententrageeinheit angeordnet ist. Damit kann insbesondere ein Oberkörper eines Patienten effizient und gezielt behandelt werden. Zudem wird vorgeschlagen, dass die zweite Behandlungseinheit in dem Unterkörperbereich der Patiententrageeinheit angeordnet ist. Dadurch kann ein Unterkörper eines Patienten effizient und gezielt behandelt werden.

Möglicherweise könnten die erste Behandlungseinheit und zumindest die zweite Behandlungseinheit auf einer selben Seite der Patiententrageeinheit, insbesondere nebeneinander, angeordnet sein. Es wäre denkbar, dass zumindest eine Seite der Patiententrageeinheit frei von zumindest einer der Behandlungseinheiten ist. Wenn die erste Behandlungseinheit und die zweite Behandlungseinheit auf unterschiedlichen Seiten der Patiententrageeinheit angeordnet sind, kann eine besonders effiziente und komfortable Behandlung eines Patienten bereitgestellt werden.

Es wäre denkbar, dass die Patiententrageeinheit eine Lage, insbesondere eine Ausrichtung und/oder eine Positionierung, des Patienten, beispielsweise durch ein Begrenzungselement, vorgibt. Das Begrenzungselement könnte den Patienten in dem Behandlungszustand zumindest teilweise umgeben und/oder einbetten. Alternativ und/oder zusätzlich könnte der Bediener Lagedaten und/oder Körperformdaten des Patienten über die Bedienerschnittstelle einstellen und/oder auswählen. Insbesondere sind die Lagedaten und/oder die Körperformdaten auf den Patienten individualisiert. Die Lagedaten könnten die Ausrichtung und/oder die Positionierung und/oder eine Relativlage zumindest eines Teils und vorzugsweise des ganzen Körpers des Patienten auf der Patiententrageeinheit beschreiben. Der Teil des Körpers könnte beispielsweise ein Arm und/oder ein Bein sein. Bei den Körperformdaten könnte es sich insbesondere um eine Ausgestaltung zumindest des Teils und vorzugsweise des ganzen Körpers des Patienten, insbesondere um Daten der Körperform, wie beispielswiese Daten einer Außenhülle und/oder einer Haut des Patienten und/oder eines Körperumfangs und/oder einer Körpergröße, handeln.

Damit eine individualisierte Behandlung eines Patienten noch weiter optimiert und vorteilhaft auf eine Lage und/oder eine Körperform eines Patienten angepasst werden kann, wird vorgeschlagen, dass die Patientenbehandlungsvorrichtung eine Erfassungseinheit zur Erfassung von Lagedaten und/oder Körperformdaten des Patienten auf der Patiententrageeinheit aufweist. Die Erfassungseinheit könnte beispielsweise eine Kameraeinheit und/oder eine Radareinheit und/oder eine Sensoreinheit aufweisen. Das Begrenzungselement könnte zumindest die Erfassungseinheit, insbesondere die Sensoreinheit, zur Erfassung von Lagedaten und/oder Körperformdaten des Patienten aufweisen. Die Patiententrageeinheit könnte einen Scanner als Erfassungseinheit aufweisen, welcher die Lagedaten und/oder Körperformdaten des Pateinten mittels eines Scanvorgangs erfasst. Vorteilhaft ist die Erfassungseinheit als eine druckempfindliche Unterlage der Patientenbehandlungsvorrichtung ausgebildet, welche in zumindest dem Behandlungszustand auf der Patiententrageeinheit angeordnet ist. Vorteilhaft ist die Erfassungseinheit dazu vorgesehen, ein zwei- und/oder dreidimensionales Modell des Patienten, insbesondere ein Körpermodell des Patienten, basierend auf den Lagedaten und/oder den Körperformdaten zu erstellen- Besonders bevorzugt könnte die Steuereinheit die Lagedaten und/oder die Körperformdaten in der Speichereinheit speichern.

In einer besonders bevorzugten Ausgestaltung der Erfindung wird vorgeschlagen, dass die Steuereinheit zur Steuerung der ersten Behandlungseinheit und/oder der zweiten Behandlungseinheit, insbesondere zur Ermittlung der Behandlungslage, die Lagedaten und/oder die Körperformdaten mit den Patientendaten verknüpft Damit kann eine Behandlung eines Patienten weiter optimiert und vorteilhaft effizienter gestaltet werden. Die Steuereinheit könnte in dem Betriebszustand die Lagedaten und/oder die Körperformdaten und die Patientendaten verarbeiten und/oder auswerten. Die Steuereinheit könnte eine Steuerung der ersten Behandlungseinheit und/oder der zweiten Behandlungseinheit an eine Auswertung der Verknüpfung der Lagedaten und/oder der Körperformdaten und der Patientendaten anpassen.

Darüber hinaus geht die Erfindung aus von einem Verfahren zur Steuerung einer Patientenbehandlungsvorrichtung nach einer der vorhergehenden Ausgestaltungen umfassend die Schritte: Einlesen von Lagedaten und/oder Körperformdaten eines Patienten; Ermitteln und/oder Einlesen von Patientendaten des Patienten; Verknüpfen der Lagedaten und/oder der Körperformdaten mit den Patientendaten; Berechnen von Bearbeitungsdaten aus den Patientendaten, den Lagedaten und/oder den Körperformdaten für eine Steuerung einer ersten Behandlungseinheit zur Behandlung eines ersten Körperbereichs des Patienten und für eine Steuerung zumindest einer zweiten Behandlungseinheit zur Behandlung zumindest eines zweiten Körperbereichs des Patienten; und Ausführen der Bearbeitungsdaten durch eine individuelle Steuerung, insbesondere gleichzeitige Steuerung, der ersten Behandlungseinheit und zumindest der zweiten Behandlungseinheit. Damit kann ein individualisiertes Verfahren zur Steuerung einer Patientenbehandlungsvorrichtung, hinsichtlich einer Behandlung eines Patienten, bereitgestellt werden. Vorteilhaft sind die Schritte des Verfahrens untereinander austauschbar, insbesondere ist eine Reihenfolge der Schritte des Verfahrens anpassbar und/oder wechselbar. Möglicherweise könnte auch zumindest einer der Schritte ausgelassen und/oder übersprungen und/oder zu einem späteren Zeitpunkt des Verfahrens hinsichtlich eines zeitlichen Verlaufs des Verfahrens, nachgeholt werden.

Des Weiteren geht die Erfindung aus von einem Computerprogrammprodukt, umfassend Befehle, die bei einer Ausführung eines Programms durch einen Computer diesen veranlassen, die Schritte des Verfahrens nach einer vorhergehenden Ausgestaltung auszuführen.

Zudem geht die Erfindung aus von einem computerlesbaren Medium, umfassend Befehle, die bei der Ausführung durch einen Computer diesen veranlassen, die Schritte des Verfahrens nach einer vorhergehenden Ausgestaltung auszuführen.

Die Patientenbehandlungsvorrichtung soll hierbei nicht auf die oben beschriebene Anwendung und Ausführungsform beschränkt sein. Insbesondere kann die Patientenbehandlungsvorrichtung zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen:

- Fig. 1: eine erfindungsgemäße Patientenbehandlungsvorrichtung und
- Fig. 2: ein Verfahren zur Steuerung der Patientenbehandlungsvorrichtung.

### Beschreibung des Ausführungsbeispiels

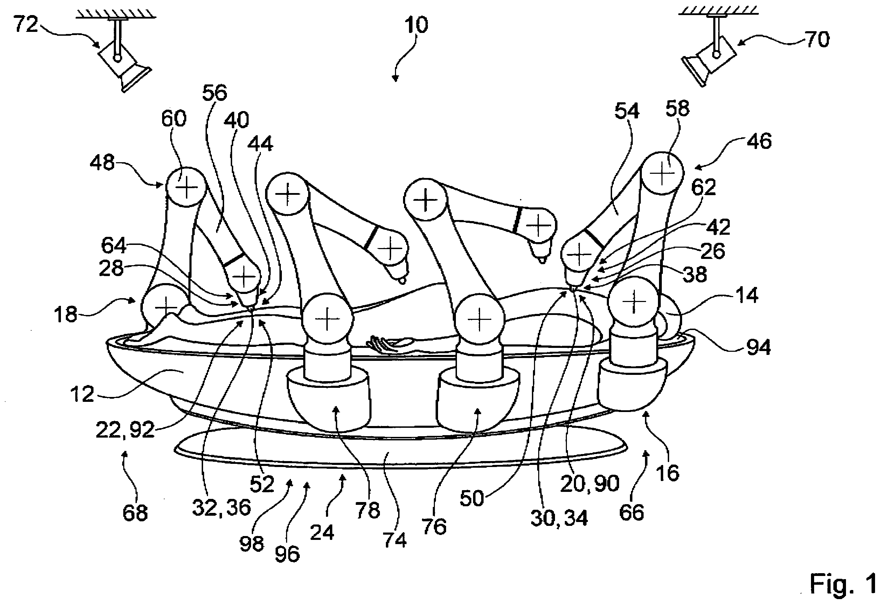

Die Figur 1 zeigt eine Patientenbehandlungsvorrichtung 10 mit einer als Liege ausgebildeten Patiententrageeinheit 12 für einen Patienten 14. Die Patientenbehandlungsvorrichtung 10 weist einen Fuß 74 zur Halterung der Patiententrageeinheit 12 auf. Vorliegend stellt die Patiententrageeinheit 12 eine längliche und/oder ovale Liegefläche 94 für den Patienten 14 bereit. In einem Behandlungszustand befindet sich der Patient 14 auf der Liegefläche 94 und liegt in dieser beispielhaften Ausführung in einer Bauchlage auf der Liegefläche 94.

Die Patientenbehandlungsvorrichtung 10 weist eine erste Behandlungseinheit 16 zur Behandlung eines ersten Körperbereichs 20 des Patienten 14 und zumindest eine zweite Behandlungseinheit 18 zur Behandlung zumindest eines zweiten Körperbereichs 22 des Patienten 14 auf. Der erste Körperbereich 20 definiert hierbei einen Rückenbereich 90 des Patienten 14 und der zweite Körperbereich 22 beispielhaft einen Beinbereich 92 des Patienten 14. Vorliegend sind die erste Behandlungseinheit 16 und die zweite Behandlungseinheit 18 auf unterschiedlichen Seiten der Patiententrageeinheit 12 angeordnet. Zu einer vereinfachteren Darstellung weist die Patientenbehandlungsvorrichtung 10 lediglich nur noch eine dritte Behandlungseinheit 76 und eine vierte Behandlungseinheit 78 auf, welche auf einer seiben Seite der Patiententrageeinheit 12 wie die erste Behandlungseinheit 16 angeordnet sind. Alternativ könnte die Patientenbehandlungsvorrichtung 10 noch weitere Behandlungseinheiten aufweisen, welche beispielsweise neben der ersten Behandlungseinheit 16 und/oder zumindest der zweiten Behandlungseinheit 18, und möglicherweise auf unterschiedlichen Seiten der Patiententrageeinheit 12, positioniert sind. Im Folgenden sind jeweils die Ausgestaltungen der ersten Behandlungseinheit 16 und der zweiten Behandlungseinheit 18 genauer beschrieben, wobei die Beschreibung ebenfalls auf zumindest die dritte Behandlungseinheit 76 und die vierte Behandlungseinheit 78 übertragbar ist.

Die erste Behandlungseinheit 16 und die zweite Behandlungseinheit 18 sind zur Behandlung eines Bewegungsapparats des Patienten 14 vorgesehen. Vorliegend ist die erste Behandlungseinheit 16 in einem Oberkörperbereich 66 der Patiententrageeinheit 12 angeordnet. In dem Behandlungszustand befindet sich ein Oberkörper, zumindest ein Kopf und/oder eine Schulter, des Patienten 14 in dem Oberkörperbereich 66 der Patiententrageeinheit 12. Zur Behandlung durch die erste Behandlungseinheit 16 befindet sich folglich der erste Körperbereich 20 des Patienten 14 in dem Oberkörperbereich 66. In dieser beispielhaften Ausführung ist die zweite Behandlungseinheit 18 in einem Unterkörperbereich 68 der Patiententrageeinheit 12 angeordnet. In dem Behandlungszustand befindet sich ein Unterkörper, beispielsweise zumindest ein Fuß und/oder ein Bein, des Patienten 14 in dem Unterkörperbereich 68. Zur Behandlung durch die zweite Behandlungseinheit 18 befindest sich der zweite Körperbereich 22 des Patienten 14 in dem Unterkörperbereich 68.

Zur Behandlung zumindest eines Teils des Bewegungsapparats des Patienten 14 im ersten Körperbereich 20 und/oder im zweiten Körperbereich 22 weisen die erste Behandlungseinheit 16 und die zweite Behandlungseinheit 18 eine Bearbeitungseinheit 26, 28 mit zumindest einem Bearbeitungskopf 30, 32 auf. Vorliegend weist die Behandlungseinheit 26, 28 einen Satz von Bearbeitungsköpfen 30, 32 auf, welche untereinander austauschbar und mit einer Kopplungseinheit 62, 64 der Behandlungseinheit 26, 28 verbindbar sind. In dieser beispielhaften Ausführung handelt es sich bei der Kopplungseinheit 62, 64 um ein Magazin, welches den Satz von Bearbeitungsköpfen 30, 32 enthält. In dem Betriebsmodus steuert eine Steuereinheit 24 einen Austausch und/oder ein Auswechseln des Bearbeitungskopfes 30, 32.

Der Satz von Bearbeitungsköpfen 30, 32 umfasst unterschiedliche Bearbeitungsköpfe 30. 32 zur Bearbeitung des Patienten 14 mit unterschiedlichen Behandlungen und/oder Therapien. In dieser beispielhaften Ausführung weist die Bearbeitungseinheit 26, 28 zumindest einen als Massagekopf 38, 40 ausgebildeten Bearbeitungskopf 30, 32 zur Durchführung einer Massage im ersten Körperbereich 20 und/oder im zweiten Körperbereich 22 auf. Vorliegend ist die Patientenbehandlungsvorrichtung 10 zur Detektion zumindest eines zu behandelnden Teils des Bewegungsapparats, vorzugsweise der Patientendaten, des Patienten 14, vorgesehen. Hierbei weist die Bearbeitungseinheit 26, 28 einen Detektionskopf 42, 44 zur Detektion zumindest des zu behandelnden Teils des Bewegungsapparats des Patienten 14 auf.

Vorliegend ist der in Figur 1 sichtbar dargestellte Bearbeitungskopf 30, 32 ein Wellentherapiekopf 34, 36 der Bearbeitungseinheit 30, 32 zur Durchführung einer Wellentherapie, vorzugsweise einer Stoßwellentherapie, im ersten Körperbereich 20 und/oder im zweiten Körperbereich 22. Der Wellentherapiekopf 34, 36 ist zur radialen Stoßwellentherapie vorgesehen. In dieser beispielhaften Ausführung ist der Wellentherapiekopf 34, 36 ebenfalls zur fokussierten Stoßwellentherapie vorgesehen.

Zur Ausrichtung und Positionierung des Bearbeitungskopfes 30, 32 weisen die erste Behandlungseinheit 16 und die zweite Behandlungseinheit 18 jeweils eine Transporteinheit 46, 48 auf, mittels welcher der Bearbeitungskopf 30, 32 In eine auf Patientendaten basierende Behandlungslage 50, 52 bringbar ist. Die Transporteinheit 46, 48 weist einen Roboterarm 54, 56 mit zumindest einem Gelenk 58, 60 auf. Der Roboterarm 54, 56 ist als ein hochsensibler Medizinal-Roboterarm ausgebildet. Vorliegend weist der Roboterarm 54, 56 mehrere Gelenke auf, welche jedoch identisch zueinander ausgebildet sind und demnach der Übersichtlichkeit halber lediglich ein Gelenk des Roboterarms 54, 56 mit einem Bezugszeichen versehen ist. Das Gelenk 58, 60 ist als ein Kugelgelenk ausgebildet.

Die Figur 1 zeigt einen Betriebszustand der Patientenbehandlungsvorrichtung 10 innerhalb des Behandlungszustands. Der Betriebszustand umfasst mehrere Betriebsmodi. In zumindest einem Betriebsmodus steuert die Steuereinheit 24 die erste Behandlungseinheit 16 und zumindest die zweite Behandlungseinheit 18 basierend auf Patientendaten des Patienten 14 Jeweils individuell und vorliegend gleichzeitig.

Der Figur 1 ist zu entnehmen, dass die Patientenbehandlungsvorrichtung 10 eine Erfassungseinheit 70 zur Erfassung von Lagedaten und/oder Körperformdaten des Patienten 14 auf der Patiententrageeinheit 12 aufweist. Beispielhaft weist die Patientenbehandlungsvorrichtung 10 eine weitere Erfassungseinheit 72 auf, welche identisch zu der Erfassungseinheit 70 ausgebildet ist Die Erfassungseinheit 70, 72 ist als eine Kamera ausgebildet. Die Patiententrageeinheit 12 könnte noch zumindest eine weitere Erfassungseinheit umfassen, welche als Scanner ausgebildet ist und den Patienten 14 zur Erfassung von Lagedaten und/oder Körperformdaten scannt. Eine Kombination mit der Erfassungseinheit 70 und der weiteren Erfassungseinheit 72 ermöglichen eine vollständige Erfassung der Lagedaten und/oder Körperformdaten.

In dieser beispielhaften Ausführung ist die Steuereinheit 24 ein Computer. Ein Computerprogrammprodukt 96 umfassend Befehle, die bei einer Ausführung eines Programms durch einen Computer, vorliegend durch die Steuereinheit 24, diesen veranlassen, Schritte eines Im folgenden Verlauf beschriebenen Verfahrens zur Steuerung der Patientenbehandlungsvorrichtung 10 auszuführen. Hierbei ist ein computerlesbares Medium 98, umfassend Befehle, dazu vorgesehen, die Befehle bei der Ausführung durch den Computer, vorliegend durch die Steuereinheit 24, diesen dazu zu veranlassen, die Schritte des Verfahrens zur Steuerung der Patientenbehandlungsvorrtchtung 10 auszuführen.

Zur Steuerung der ersten Behandlungseinheit 16 und der zweiten Behandlungseinheit 18 verknüpft die Steuereinheit 24 die Lagedaten und/oder die Körperformdaten mit den Patientendaten.

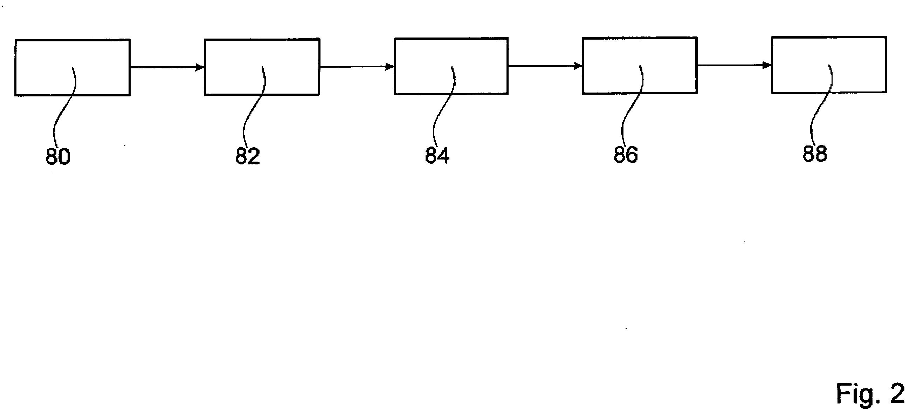

Ein in Figur 2 dargestelltes Ablaufdiagramm des Verfahrens zur Steuerung der Patientenbehandlungsvorrichtung 10 zeigt mehrere Verfahrensschritte des Verfahrens. Hierbei wird hinsichtlich eines zeitlichen Verlaufs nach einem ersten Verfahrensschritt 80 des Verfahrens ein zweiter Verfahrensschritt 82 des Verfahrens, dann ein dritter Verfahrensschritt 84 des Verfahrens, dann ein vierter Verfahrensschritt 86 des Verfahrens und schlussendlich ein fünfter Verfahrensschritt 88 des Verfahrens durchgeführt.

In dem ersten Verfahrensschritt 80 werden die Lagedaten und/oder die Körperformdaten des Patienten 14 eingelesen. In dem zweiten Verfahrensschritt 82 werden die Patientendaten des Patienten 14 eingelesen. Eine Verknüpfung der Lagedaten und/oder der Körperformdaten und der Patientendaten erfolgt in dem dritten Verfahrensschritt 84. Anschließend werden in dem vierten Verfahrensschritt 86 Bearbeitungsdaten aus den Patientendaten und den Lagedaten und/oder den Körperformdaten für eine Steuerung der ersten Behandlungseinheit 16 zur Behandlung des ersten Körperbereichs 20 des Patienten 14 und für eine Steuerung zumindest der zweiten Behandlungseinheit 18 zur Behandlung zumindest des zweiten Körperbereichs 22 des Patienten 14 berechnet. In dem fünften Verfahrensschritt 88 werden die Bearbeitungsdaten durch eine individuelle Steuerung der ersten Behandlungseinheit 16 und der zweiten Behandlungseinheit 18, vorliegend gleichzeitig, ausgeführt.

### Bezugszeichen

- 10: Patientenbehandlungsvorrichtung
- 12: Patiententrageeinheit
- 14: Patient
- 16: Behandlungseinheit
- 18: Behandlungseinheit
- 20: Körperbereich
- 22: Körperbereich
- 24: Steuereinheit
- 26: Bearbeitungseinheit
- 28: Bearbeitungseinheit
- 30: Bearbeitungskopf
- 32: Bearbeitungskopf
- 34: Wellentherapiekopf
- 36: Wellentherapiekopf
- 38: Massagekopf
- 40: Massagekopf
- 42: Detektionskopf
- 44: Detektionskopf
- 46: Transporteinheit
- 48: Transporteinheit
- 50: Behandlungslage
- 52: Behandlungslage
- 54: Roboterarm
- 56: Roboterarm
- 58: Gelenk
- 60: Gelenk
- 62: Kopplungseinheit
- 64: Kopplungseinheit
- 66: Oberkörperbereich
- 68: Unterkörperbereich
- 70: Erfassungseinheit
- 72: Erfassungseinheit
- 74: Fuß
- 76: Behandlungseinheit
- 78: Behandlungseinheit
- 80: Verfahrensschritt
- 82: Verfahrensschritt
- 84: Verfahrensschritt
- 86: Verfahrensschritt
- 88: Verfahrensschritt
- 90: Rückenbereich
- 92: Beinbereich
- 94: Liegefläche
- 96: Computerprogrammprodukt
- 98: Computerlesbares Medium

## Patentansprüche

1. Patientenbehandlungsvorrichtung (10) mit einer Patiententrageeinheit (12) für einen Patienten (14), mit einer ersten Behandlungseinheit (16) zur Behandlung eines ersten Körperbereichs (20) des Patienten (14) und mit zumindest einer zweiten Behandlungseinheit (18) zur Behandlung zumindest eines zweiten Körperbereichs (22) des Patienten (14), **gekennzeichnet durch** eine Steuereinheit (24), welche in zumindest einem Betriebsmodus die erste Behandlungseinheit (16) und zumindest die zweite Behandlungseinheit (18), basierend auf Patientendaten des Patienten (14) jeweils individuell steuert.

2. Patientenbehandlungsvorrichtung (10) nach Anspruch 1, **dadurch gekennzeichnet, dass** die erste Behandlungseinheit (16) und/oder die zweite Behandlungseinheit (18) zur Behandlung zumindest eines Teils eines Bewegungsapparats des Patienten im ersten Körperbereich (20) und/oder im zweiten Körperbereich (22) eine Bearbeitungseinheit (26, 28) mit zumindest einem Bearbeitungskopf (30. 32) aufweist/aufweisen.

3. Patientenbehandlungsvorrichtung (10) nach Anspruch 2, **dadurch gekennzeichnet, dass** die Bearbeitungseinheit (26, 28) zumindest einen Wellentherapiekopf (34, 36) zur Durchführung einer Wellentherapie, insbesondere einer Stoßwellentherapie, im ersten Körperbereich (20) und/oder im zweiten Körperbereich (22) aufweist.

4. Patientenbehandlungsvorrichtung (10) nach Anspruch 3, **dadurch gekennzeichnet, dass** der Wellentherapiekopf (34, 36) zur radialen Stoßwellentherapie vorgesehen ist.

5. Patientenbehandlungsvorrichtung (10) nach Anspruch 3 oder 4, **dadurch gekennzeichnet, dass** der Wellentherapiekopf (34. 36) zur fokussierten Stoßwellentherapie vorgesehen ist.

6. Patientenbehandlungsvorrichtung (10) nach einem der Ansprüche 2 bis 5, **dadurch gekennzeichnet, dass** die Bearbeitungseinheit (26, 28) zumindest einen Massagekopf (36, 40) zur Durchführung einer Massage im ersten Körperbereich (20) und/oder Im zweiten Körperbereich (22) aufweist.

7. Patientenbehandlungsvorrichtung (10) nach einem der Ansprüche 2 bis 6, **dadurch gekennzeichnet, dass** die Bearbeitungseinheit (26, 28) einen Detektionskopf (42, 44) zur Detektion zumindest eines zu behandeinden Teils eines Bewegungsapparats, insbesondere eines latenten Schmerzpunkts, des Patienten (14) aufweist.

8. Patientenbehandlungsvorrichtung (10) nach einem der Ansprüche 2 bis 7, **dadurch gekennzeichnet, dass** die erste Behandlungseinheit (16) und/oder die zweite Behandlungseinheit (18) eine Transporteinheit (46, 48) aufweist/aufweisen, mittels welcher zumindest der Bearbeitungskopf (30, 32) in eine auf den Patientendaten basierende Behandlungslage (50, 52) bringbar ist.

9. Patientenbehandlungsvorrichtung (10) nach Anspruch 8, **dadurch gekennzeichnet, dass** die Transporteinheit (46, 48) einen Roboterarm (54, 56) mit zumindest einem Gelenk (58, 60) aufweist.

10. Patientenbehandlungsvorrichtung (10) nach einem der Ansprüche 2 bis 9, **dadurch gekennzeichnet, dass** die Bearbeitungseinheit (26, 28) eine Kopplungseinheit (62, 64) und einen Satz von Bearbeitungsköpfen (30, 32) aufweist, welche untereinander austauschbar und mit der Kopplungseinheit (62, 64) verbindbar sind.

11. Patientenbehandlungsvorrichtung (10) nach einem der vorhergehenden Ansprüche, **dadurch gekennzeichnet, dass** die erste Behandlungseinheit (16) in einem Oberkörperbereich (66) der Patiententrageeinheit (12) angeordnet ist.

12. Patientenbehandlungsvorrichtung (10) nach einem der vorhergehenden Ansprüche, **dadurch gekennzeichnet, dass** die zweite Behandlungseinheit (18) in einem Unterkörperbereich (68) der Patiententrageeinheit (12) angeordnet ist.

13. Patientenbehandlungsvorrichtung (10) nach einem der vorhergehenden Ansprüche, **dadurch gekennzeichnet, dass** die erste Behandlungseinheit (16) und die zweite Behandlungseinheit (18) auf unterschiedlichen Seiten der Patiententrageeinheit (12) angeordnet sind.

14. Patientenbehandlungsvorrichtung (10) nach einem der vorhergehenden Ansprüche, **gekennzeichnet durch** eine Erfassungseinheit (70) zur Erfassung von Lagedaten und/oder Körperformdaten des Patienten (14) auf der Patiententrageeinheit (12).

15. Patientenbehandlungsvorrichtung (10) nach Anspruch 14, **dadurch gekennzeichnet, dass** die Steuereinheit (24) zur Steuerung der ersten Behandlungseinheit (16) und/oder der zweiten Behandlungseinheit (18) die Lagedaten und/oder die Körperformdaten mit den Patientendaten verknüpft.

16. Verfahren zur Steuerung einer Patientenbehandlungsvorrichtung (10), umfassend die Schritte:
Einlesen von Lagedaten und/oder Körperformdaten eines Patienten (14);
Ermitteln und/oder Einlesen von Patientendaten des Patienten (14);
Verknüpfen der Lagedaten und/oder der Körperformdaten mit den Patientendaten;
Berechnen von Bearbeitungsdaten aus den Patientendaten, den Lagedaten und/oder den Körperformdaten für eine Steuerung einer ersten Behandlungseinheit (16) zur Behandlung eines ersten Körperbereichs (20) des Patienten (14) und für eine Steuerung zumindest einer zweiten Behandlungseinheit (18) zur Behandlung zumindest eines zweiten Körperbereichs (22) des Patienten (14); und
Ausführen der Bearbeitungsdaten durch eine individuelle Steuerung, insbesondere gleichzeitige Steuerung, der ersten Behandlungseinheit (16) und zumindest der zweiten Behandlungseinheit (18).

17. Computerprogrammprodukt umfassend Befehle, die bei einer Ausführung eines Programms durch einen Computer diesen veranlassen, die Schritte des Verfahrens nach Anspruch 16 auszuführen.

18. Computerlesbares Medium umfassend Befehle, die bei der Ausführung durch einen Computer diesen veranlassen, die Schritte des Verfahrens nach Anspruch 16 auszuführen.
